# EUROPÄISCHE PATENTSCHRIFT

(11) **EP 1 440 942 B1**
(45) Veröffentlichungstag und Bekanntmachung des Hinweises auf die Patenterteilung: **05.10.2005**
(21) Anmeldenummer: 04008187.9
(22) Anmeldetag: 08.11.2002
(51) Int. Cl.: C02F 1/34

(54) **Vorrichtung zur Behandlung von Abwasser, Klärschlamm und organischen Substraten**
Device for the treatment of effluent, sludge and organic substrates
Dispositif de traitement de substrats organiques, de boues résiduaires et d'eaux usées

(30) Priorität: 12.11.2001 DE 10155161
(43) Veröffentlichungstag der Anmeldung: 28.07.2004
(62) Teilanmeldung aus: 02781155.3
(73) Patentinhaber: Emu Unterwasserpumpen Gmbh, 95030 Hof (DE)
(72) Erfinder: Schmid, Andreas, Dr., 95213 Münchberg (DE); Geier, Wolfgang, 95030 Hof (DE)
(74) Vertreter: LOUIS, PÖHLAU, LOHRENTZ

(56) Entgegenhaltungen:
- DE-A- 4 114 694
- GB-A- 1 589 551
- US-A- 4 341 632
- US-A- 5 494 585
- SCHMELZ K-G, WINTER A: "Klärschlammverminderung durch verschiedene Methoden der Desintegration auf der Kläranlage Schermbeck" 18. BOCHUMER WORKSHOP SIEDLUNGSWASSERWIRTSCHAFT: INNOVATIONEN IN DER ABWASSERBESEITIGUNG, SCHRIFTENREIHE SIEDLUNGSWASSERWIRTSCHAFT BOCHUM, 21. September 2000 (2000-09-21), Seiten 205-224, XP002231503
- MÜLLER ET AL: "Verfahren und Anwendungsgebiete der mechanischen Klärschlammdesintegration" KA-WASSERWIRTSCHAFT, ABWASSER, ABFALL, Bd. 47, Nr. 4, 1. April 2000 (2000-04-01), Seiten 570-576, XP002231544

## Beschreibung

Die Erfindung betrifft eine Vorrichtung zur Behandlung von fädige und flockenbildende Mikroorganismen-haltigem Abwasser, Klärschlamm oder in Biogasanlagen verwendeten organischen Substraten zur Verringerung oder Vermeidung der Bildung von Blähschlamm, Schwimmschlamm und/oder Schaum.

Bei Kläranlagen mit biologischen Reinigungsstufen kommt es immer wieder zu Betriebsproblemen, die auf nicht sedimentierende bzw. flotierende Schlammaggregate insbesondere im Belebungsbecken und im Nachklärbecken zurückzuführen sind.

Für einen stabilen Betrieb einer Kläranlage ist es erforderlich, daß die zur Reinigung des Abwassers verwendete Biomasse, die die zur Reinigung verwendeten Mikroorganismen umfaßt, von dem gereinigten Abwasser getrennt wird. Bei dem Belebungsverfahren wird die Biomasse in der Regel in einem Nachklärbecken durch Sedimentation abgetrennt und teilweise oder vollständig zum Belebungsbecken zurückgeführt. Ein störungsfreier Betrieb einer Kläranlage mit biologischer Reinigungsstufe erfordert daher eine gut sedimentierende Biomasse bzw. einen gut sedimentierenden Belebtschlamm.

Die in dem Belebungsbecken verwendete Biomasse ist eine Mischbiozönose verschiedenster Mikroorganismen. In der Mischbiozönose sind unter anderem fädige Bakterien bzw. Fadenbakterien und flockenbildende Bakterien enthalten.

Zu einer Entstehung von nicht sedimentierenden oder aufschwimmenden Schlammaggregaten kommt es bei einem vermehrten Wachstum von fädigen Bakterien, die dann zu einem Fadengeflecht aggregieren. Dieses Fadengeflecht behindert den Eindick- und Absetzvorgang des belebten Schlamms erheblich. Die aufschwimmenden Schlammaggregate bestehen überwiegend aus Blähschlamm, Schwimmschlamm und Schaum.

Blähschlamm, Schwimmschlamm und Schaum können weiterhin entstehen, wenn in dem Abwasser fein verteilte Gasbläschen, hydrophobe Abwasserinhaltsstoffe und/oder Zellstrukturen sowie oberflächenaktive Substanzen enthalten sind.

Ein stabiler Betrieb einer biologischen Kläranlage ist bei einer Bildung von Blähschlamm, Schwimmschlamm oder Schaum sehr erschwert bzw. nicht möglich.

Aus der DE 198 43 862 A1 ist ein Verfahren zur Verhinderung der Schwimm-und/oder Blähschlammbildung in Kläranlagen bekannt, bei dem der biomassehaltige Schlamm mit Ultraschall behandelt wird.

Aus der DE 195 17 381 C1 ist eine weitere Einrichtung zum Zerstören zellulärer Strukturen in Schlämmen biologischer Kläranlagen unter Verwendung einer Ultraschallbeaufschlagung bekannt.

Nachteilig ist, daß diese Verfahren in technischer Hinsicht aufwendig sind und an das Bedienungspersonal erhöhte Anforderungen gestellt werden.

In KA-Wasserwirtschaft, Abwasser, Abfall 2001 (48) Nr. 5, Seiten 598 bis 604 wird der Einsatz von Füll- und Flockungsmitteln auf Poly-Aluminium-Basis, wie bspw. Poly-Aluminiumhydroxid oder Poly-Aluminiumchloride, diskutiert. Die Verwendung solcher Füll- und Flockungsmittel ist jedoch mit erheblichen Mehrkosten verbunden.

In WAP (Wasser Abwasser Praxis) 3/99 Seiten 25 bis 31 wird offenbart, daß durch mechanische Zerkleinerung von Bläh- und Schwimmschlämmen kleinere Aggregate erzeugt werden, die verbesserte Sedimentationseigenschaften aufweisen.

Die Zerkleinerung des Bläh- und Schwimmschlammes erfolgt unter Verwendung einer Rührwerkskugelmühle, einem Scherspalt-, einem Ultraschall- und einem Hochdruckhomogenisator. Um eine wirksame Behandlung des Schwimmschlamms durchführen zu können, ist ein Energieeintrag von wenigstens etwa 1000 kJ/m³ Schlamm erforderlich.

Nachteilig ist, daß die in dieser Veröffentlichung vorgeschlagenen Zerkleinerungsgeräte aufwendig und kostenintensiv sind und sich daher insbesondere für viele kleine kommunale Kläranlagen nicht eignen.

Aus SCHMELZ K-G, WINTER A: 'Klärschlammverminderung durch verschiedene Methoden der Desintegration auf der Kläranlage Schermbeck' 18. BOCHUMER WORKSHOP SIEDLUNGSWASSERWIRTSCHAFT: INNOVATIONEN IN DER ABWASSERBESEITIGUNG, SCHRIFTENREIHE SIEDLUNGSWASSERWIRTSCHAFT BOCHUM, 21.09.00, Seiten 205-224, ist ein HochdruckHomogenisator bekannt, der zu einer Zerstörung der Zellwände der im behandelten Abwasser enthaltenen Mikororganismen, d.h. sowohl fädigen als auch flockenbildenden Mikroorganismen führt.

Aufgabe der Erfindung ist es, eine Vorrichtung bereitzustellen, die einfach in der Handhabung und preiswert im Betrieb ist sowie wirksam die Bildung von nicht sedimentierenden Schlammaggregaten in Kläranlagen, Biogasreaktoren etc. verringert oder verhindert.

Die der Erfindung zugrundeliegende Aufgabe wird durch eine Vorrichtung gemäß Anspruch 1 gelöst.

Nachstehend wird die Erfindung im wesentlichen unter Bezug auf Kläranlagen beschrieben. Die Erfindung ist jedoch keinesfalls auf Kläranlagen beschränkt. Vielmehr kann die Erfindung in weiteren biologischen Anlagen verwendet werden, in denen fädige und flockenbildende Mikroorganismen in einer Mischbiozönose vorliegen und zusammenwirken. Die Erfindung ist beispielsweise auch bei Biogasreaktoren und Anaerobfermentern verwendbar. Insofern gelten die nachstehenden Ausführungen entsprechend für Biogasreaktoren und Anaerobfermenter.

Das Abwasser ist im Sinne der Erfindung bevorzugt ein Belebtschlamm oder eine Mischbiozönose in biologischen Kläranlagen.

Gemäß der vorliegenden Erfindung kann das seit Jahrzehnten bestehende Problem der Entstehung von Blähschlamm, Schwimmschlamm und Schaum in biologischen Reinigungsstufen von Kläranlagen auf überraschend einfache Art und Weise gelöst werden.

Gemäß der vorliegenden Erfindung wird das Abwasser, bevorzugt Belebtschlamm oder eine Mischbiozönose, in einer Rohrleitung einer Scherbeanspruchung durch hydrodynamisch erzeugte Scherfelder unterworfen. Die Scherfelder entstehen durch im in der Rohrleitung geführten Abwasser, bevorzugt Belebtschlamm oder eine Mischbiozönose, auftretende Geschwindigkeitsgradienten.

Die Scherbeanspruchung führt zu einer Zerkleinerung bzw. Zerteilung von aus langfädigen Mikroorganismen gebildeten Schlammaggregaten oder Fadengeflechten. Weiterhin werden in diesen Schlammaggregaten eingeschlossene oder anhaftende fein verteilte Gasblasen freigesetzt. Diese in den unbehandelten Schlammaggregaten enthaltenen Gasblasen tragen zu einer Verschlechterung des Sedimentationsverhaltens der Schlammaggregate bei.

Schließlich kommt es durch die Scherbeanspruchung zu einer teilweisen Zerstörung der fädigen Bakterien und zu einer Verkürzung der Filamentlänge der fädigen Bakterien.

Der auf die in der Mischbiozönose enthaltenen fädigen Bakterien durch das erzeugte Scherfeld ausgeübte Streß führt insbesondere zu Veränderungen im Metabolismus der fädigen Bakterien, was zu einer Veränderung der Wachstumskinetik bei den fädigen Mikroorganismen bzw. Bakterien führt. Das Wachstum der fädigen Mikroorganismen wird deutlich verringert.

Die Veränderung im Metabolismus von fädigen Mikroorganismen ist insbesondere auf die Stimulierung von Schutzmechanismen gegenüber dem durch die angelegte Scherbeanspruchung erzeugten Streß zurückzuführen. Dies führt u.a. zu einer Sekretion von extrazellulären polymeren Substanzen, die auch als EPS bezeichnet werden, vorwiegend durch die fädigen Mikroorganismen. Die sekretierten extrazellulären polymeren Substanzen bewirken vorteilhaft eine Adhäsion der im Scherfeld zerkleinerten Schlammaggregate. Diese Adhäsion wirkt der Entstehung zu kleiner Schlammpartikel entgegen, die nicht oder zu langsam sedimentieren, und führt zu einer vorteilhafte Flockung und mithin zu der gewünschten Sedimentation der Mischbiozönose.

Die relativen Anteile von flockenbildenden Mikroorganismen zu fädigen Mikroorganismen in der Mischbiozönose bzw. dem Klärschlamm ändern sich zugunsten der flockenbildenden Mikroorganismen. Ein Erhöhung des Verhältnisses von flockenbildenden Bakterien zu fädigen Bakterien in der Mischbiozönose begünstigt eine Sedimentation der Schlammaggregate.

Flockenbildende Mikroorganismen zeigen im Unterschied zu fädigen Mikroorganismen ein gutes Sedimentationsverhalten.

Bevorzugt wird die Scherbeanspruchung durch eine turbulente Strömung in der Rohrleitung erzeugt. Weiterhin ist bevorzugt, daß in dem Strömungskanal wenigstens eine Düse angeordnet ist, durch die das Abwasser geführt wird.

Das in der Rohrleitung geführte Abwasser ist hochturbulent. Die Reynoldszahl des turbulenten Abwassers bzw. der Mischbiozönose beträgt bevorzugt wenigsten 100.000, weiter bevorzugt wenigstens 180.000, noch weiter bevorzugt wenigstens 250.000, noch bevorzugter wenigstens 500.000.

Das Abwasser, bevorzugt Belebtschlamm oder eine Mischbiozönose, kann mit Hilfe einer herkömmlichen Pumpe durch die Rohrleitung gepumpt werden. Die wenigstens eine Düse kann dabei sowohl druck- als auch saugseitig im Strömungskanal angeordnet sein.

Bei der erfindungsgemäßen Vorrichtung wird die Fließgeschwindigkeit des Abwassers bei Durchtritt durch die Düse in der Rohrleitung unter abnehmenden Druck erhöht, bis Dampfdruck erreicht wird, was sich in Form von Kavitation ausbildet.

Als Kavitation wird die Entstehung von Hohlräumen in Flüssigkeiten und deren Bewegung bezeichnet. Der Hohlraum kann durch eine lokale Unterdruckphase, Scherspannungen oder einen Energieeintrag in die Flüssigkeit gerissen werden. In die so entstandenen Blasen diffundieren die in der Flüssigkeit gelösten Gase hinein. Wenn der Druck in der Flüssigkeit wieder ansteigt, kollabieren die Gasblasen durch den äußeren Druck. Während des Blasenkollaps wird der Blaseninhalt so stark komprimiert, daß hohe Drücke, bis zu einigen 1000 bar, und hohe Temperaturen, bis zu mehreren 1000 K, lokal erzeugt werden. Das Gas wird in der Blase ionisiert und Radikale entstehen.

Des weiteren emittiert die Blase während des Kollaps Schockwellen und, sofern sie sich in der Nähe von festen Oberflächen befindet, einen Flüssigkeitsjet, der durch die Blase hindurchtritt. Die von den Blasen emittierten Schockwellen und Flüssigkeitsjets haben die Kraft, um Partikelagglomerate, wie beispielsweise Schlammaggregate, aufzulösen sowie Makromoleküle und bakterielle Zellwände aufzubrechen.

In der in der Rohrleitung angeordneten Düse nimmt die Fließgeschwindigkeit zu und der Druck ab. Unter diesen Bedingungen entsteht die bei der erfindungsgemäßen Vorrichtung erwünschte Kavitation.

Beim Betrieb der erfindungsgemäßen Vorrichtung kommt es bei einer Ausführungsform bevorzugt zu einem gemeinsamen Auftreten von Scherbeanspruchung und Kavitation.

Die bspw. durch eine hochturbulente Strömung erzeugte Scherbeanspruchung sowie die durch die Düse in der Rohrleitung vorzugsweise erzeugte Kavitation wirken beim Betrieb der erfindungsgemäßen Vorrichtung äußerst vorteilhaft zusammen.

Die Scherbeanspruchung und die Kavitation wirken sich insbesondere auf die größeren fädigen Mikroorganismen bzw. auf die von fädigen Mikroorganismen gebildeten Fadengeflechte aus. Die fädigen Mikroorganismen werden teilweise durch Aufbrechen der Zellmembranen zerstört. Zu einem wesentlichen Teil kommt es zu einer Verkürzung der Filamente und somit zu einem Aufbrechen bzw. einer Zerkleinerung der Fadengeflechte.

Überraschenderweise werden die flockenbildenden Bakterien beim Betrieb der erfindungsgemäßen Vorrichtung nicht zerstört. Ferner kommt es zu keiner wesentlichen Änderung im Zellmetabolismus, so daß die Wachstumskinetik der flockenbildenden Mikroorganismen nicht wesentlich beeinträchtigt bzw. nicht wesentlich verändert wird. Somit führt der Betrieb der erfindungsgemäßen Vorrichtung im Hinblick auf die unterschiedlichen Wachstumskinetiken von fädigen und flockenbildenden Mikroorganismen zu einer vorteilhaften Veränderung der Zusammensetzung der Mischbiozönose. Diese beim Betrieb der erfindungsgemäßen Vorrichtung bewirkte Veränderung der bakteriellen Zusammensetzung führt mithin zu einer Gesundung des Klärschlamms.

Gemäß einer weiteren Ausführungsform der Erfindung ist die Dauer der Kavitation und damit die Einwirkdauer der Kavitation auf das Abwasser, bzw. die Mischbiozönose einstellbar.

Es wurde überraschenderweise herausgefunden, daß die durch die Düse in der Rohrleitung hervorgerufene Kavitation erhalten werden kann, wenn der Innendurchmesser der Rohrleitung, der in bezug auf die Fließrichtung des Abwassers, nach der Düse angeordnet ist, größer ist als der kleinste Innendurchmesser der Düse in der Rohrleitung, aber kleiner ist als der Innendurchmesser der Rohrleitung, die vor der Düse angeordnet ist.

Über die Länge der nach der Düse angeordneten Rohrleitung, kann die Dauer der Kavitation gesteuert werden. Wenn die nach der Düse angeordnete Rohrleitung verlängert wird, verlängert sich auch die Einwirkdauer auf das Abwasser, bevorzugt den Belebtschlamm oder die Mischbiozönose.

Das Verhältnis des Innendurchmessers der Rohrleitung vor der Düse, bezogen auf die Fließrichtung des Abwassers, zu dem Innendurchmesser der Rohrleitung nach der Düse kann bspw. etwa 5:1 bis etwa 1,2:1, bevorzugter 3:1 bis 1,5:1, sehr bevorzugt 2:1, betragen.

Das Verhältnis des kleinsten Innendurchmessers der Düse zu dem Innendurchmesser der in bezug auf die Düse abströmseitig angeordneten Rohrleitung kann beispielsweise 1:1,2 bis 1:3, bevorzugt 1:2, betragen.

Äußerst überraschend ist, daß durch die erzeugte Kavitation beim Betrieb der erfindungsgemäßen Vorrichtung weder an der Düse in der Rohrleitung noch an der in Fließrichtung des Abwassers nachgeordneten Rohrleitung mit gegebenenfalls angeordneten Armaturen Abrasion verursacht wird.

Es wird vermutet, daß die Kavitationsblasen aufgrund eines höheren Drucks an der Wandung des Strömungskanals überwiegend in der Mitte des Strömungskanals auftritt. Die in der Mitte der Abwasserströmung auftretenden Kavitationsblasen kommen mithin nicht in Kontakt mit der Wandung des Strömungskanals und können insoweit zu keiner Abrasion führen.

Die in der Rohrleitung vorgesehene Düse ist in der Geometrie einer Lavaldüse ausgebildet.

Bevorzugte Weiterbildungen der Vorrichtungen sind in den Unteransprüchen 2 bis 12 angegeben.

In bezug auf die Größenverhältnisse der Innendurchmesser der Rohrleitungen an der Düse der Rohrleitungen bzw. vor und nach der Düse wird auf die obigen Ausführungen verwiesen.

Es ist bevorzugt, daß die wenigstens eine Düse einen Abschnitt mit konvergierendem und divergierendem Innendurchmesser umfaßt, wobei, bezogen auf die Fließrichtung des Abwassers, bevorzugt Belebtschlamm oder eine Mischbiozönose, der Abschnitt mit konvergierendem Innendurchmesser vor dem Abschnitt mit divergierendem Innendurchmesser angeordnet ist.

Der Rohrleitung kann sich bspw., bezogen auf die Fließrichtung des Abwassers, zunächst konisch verengen bis zu einem geringsten Innendurchmesser und nachfolgend konisch erweitern. Die Düse verbindet bevorzugt zwei Rohrleitungen miteinander.

Bei einer weiteren Ausführungsform der Erfindung ist die Düse so ausgebildet, daß die Länge des Abschnitts mit konvergierendem Innendurchmesser kürzer ist als die Länge des Abschnitts mit divergierendem Innendurchmesser.

Bei dieser Ausführungsform ist die geometrische Ausgestaltung der Düse mithin nicht symmetrisch zu dem Abschnitt mit dem geringsten Innendurchmesser der Düse.

Es hat sich gezeigt, daß bei asymmetrischer Ausgestaltung der Düse die gewünschte Kavitation so eingestellt werden kann, daß eine für den Zweck der Vermeidung von Blähschlamm, Schwimmschlamm und/oder Schaum geeignete Kavitation erzeugt wird. Insbesondere läßt sich über den Austrittswinkel der Düse die Länge der nachfolgenden Kavitationsbildung und mithin die Einwirkdauer der Kavitation auf das Abwasser steuern.

Die Düse weist dabei die Geometrie einer Lavaldüse auf.

Bei einer weiteren Ausführungsform der erfindungsgemäßen Vorrichtung ist abströmseitig von der Düse in der Rohrleitung eine Drossel, beispielsweise ein Schieber, angeordnet. Über diese Drossel kann das Auftreten von Kavitation zusätzlich gesteuert werden.

In die Rohrleitung eintretende Gasblasen können einen dämpfenden Effekt auf die Kavitationsbildung ausüben. Insofern wird gemäß einer bevorzugten Ausführungsform der Anteil von Gasblasen im Abwasser, das in die Rohrleitung eingebracht wird, verringert. Bevorzugt wird das Abwasser während des Förderns in die Rohrleitung entgast, beispielsweise durch Verwendung einer Pumpe, bevorzugt einer Tauchmotorpumpe, in einem getauchten Schacht.

Die Erfindung wird nachfolgend anhand der Figuren 1 bis 8 weiter veranschaulicht. Dabei zeigt
Figur 1 eine schematische Darstellung eines beispielhaften Aufbaus einer erfindungsgemäßen Vorrichtung,
Figur 2 eine schematische Darstellung der Entgasung von Schlammaggregaten im Scherfeld,
Figur 3 eine schematische Darstellung einer durch Sekretion von extrazellulären polymeren Substanzen (EPS) bewirkten verbesserten Flockung von zerkleinerten Schlammaggregaten,
Figur 4 eine schematische Darstellung einer Verkürzung der Filamente von fädigen Mikroorganismen im Scherfeld und gegebenenfalls durch Kavitation,
Figur 5 eine schematische Darstellung des Einflusses des Betriebs der erfindungsgemäßen Vorrichtung auf die Wachstumskinetik von flockenbildenden Mikroorganismen und fädigen Mikroorganismen,
Figur 6 eine Darstellung von Meßwerten des Schlammvolumenindexes (ISV) vor und nach Anwendung der erfindungsgemäßen Vorrichtung in einer Versuchskläranlage,
Figur 7 eine Darstellung von Meßwerten, die eine Stabilisierung der Prozeßparameter durch Verwendung der erfindungsgemäßen Vorrichtung in einer Versuchskläranlage zeigen, und
Figur 8 eine schematische Darstellung einer in einem getauchten Schacht angeordneten Pumpe.

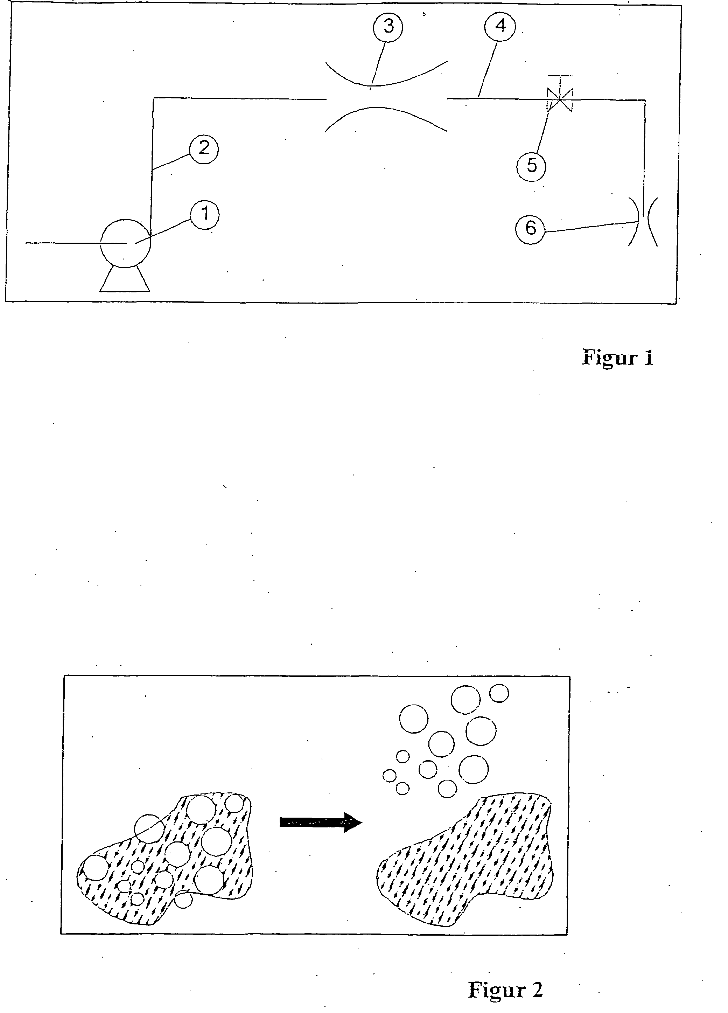

Figur 1 zeigt eine schematische Darstellung der erfindungsgemäßen Vorrichtung. Die Pumpe (1) fördert Abwasser, bevorzugt Belebtschlamm oder eine Mischbiozönose, bspw. aus einem Belebungsbecken oder einem Nachklärbecken (nicht gezeigt), über eine Rohrleitung (2) durch eine Düse (3) in eine Rohrleitung (4). In der Rohrleitung (4) kann eine Drossel (5) vorgesehen sein. Das behandelte Abwasser gelangt nachfolgend aus der Rohrleitung (4) über eine Abgabevorrichtung (6) vorzugsweise zurück in das Entnahmebecken, d.h. bspw. das Belebungsbecken bzw. das Nachklärbecken. Selbstverständlich kann das behandelte Abwasser über die Abgabevorrichtung (6) auch in ein weiteres Auffangbecken abgegeben werden.

Die Pumpe (1) kann sowohl vor der Düse (3) als auch nach der Düse (3) in der Rohrleitung(2) oder (4) angeordnet sein. Die Rohrleitung (2) weist einen Innendurchmesser auf, der bevorzugt größer als der Innendurchmesser der Rohrleitung (4) ist, wobei der Innendurchmesser der Rohrleitung (4) größer als der geringste Innendurchmesser der Düse (3) ist.

Als Pumpe (1) kann jede für die Verwendung in einem Klärbecken geeignete Pumpe verwendet werden. Die von der Düse (3) abströmseitig angeordnete Rohrleitung (4) bewirkt aufgrund des in bezug auf die anströmseitig zur Düse (3) angeordnete Rohrleitung (2) verringerten Innendurchmessers eine verlängerte Einwirkdauer der in der Düse (3) erzeugten Kavitation auf das Abwasser bzw. die Mischbiozönose. Die Länge der Rohrleitung (4) kann in Abhängigkeit von der gewünschten Einwirkdauer der Kavitation eingestellt werden. Bspw. kann die Länge der Rohrleitung (4) variabel einstellbar sein. Hierdurch kann eine gewünschte Verkürzung der Filamentlänge fädiger Bakterien als auch eine zuverlässige Freisetzung der in dem durch die fädigen Mikroorganismen erzeugten Fadengeflecht eingeschlossenen Gasblasen erreicht werden. Weiterhin wird durch den an die fädigen Mikroorganismen angelegten Streß deren Wachstum verlangsamt.

Die Einwirkdauer der Kavitation kann u.a. über die Länge der abströmseitig von der Düse (3) angeordneten Rohrleitung (4) gesteuert werden. Weiterhin kann die Entstehung von Kavitationsblasen auch durch den durch die Drosselklappe (5) einstellbaren Druck in der Rohrleitung (4) gesteuert werden. Die Abgabevorrichtung (6), die bevorzugt als Sprühdüse ausgebildet ist, bewirkt bei Abgabe der behandelten Mischbiozönose aus der erfindungsgemäßen Vorrichtung eine Entweichung der aus den Schlammaggregaten gelösten Gasblasen.

Vor der Düse (3) kann ein Konfusor angeordnet sein, der den Innendurchmesser der das Abwasser zuführenden Rohrleitung (2), d.h. der zur Düse anströmseitig angeordneten Rohrleitung (2), reduziert. Hierdurch wird der Übergang zwischen zuführender Rohrleitung (2) und Düse (3) verbessert.

Weiterhin ist es bevorzugt, zwischen Düse (3) und Drosselklappe (5) einen Diffusor anzuordnen, der den Innendurchmesser der von der Düse (3) abströmseitig angeordneten Rohrleitung (4) erweitert. Durch die Erweiterung des Innendurchmessers der Rohrleitung (4) wird der Druck in dem Abwasser, bevorzugt Belebtschlamm oder eine Mischbiozönose, erhöht und die Kavitation beendet. Dadurch kann ein Einwirken von Kavitation, d.h. ein Auftreffen von Kavitationsblasen auf die Drosselklappe (5) und mithin Schäden. bspw. Abrasion an der Drosselklappe (5) zuverlässig verhindert werden.

Bevorzugt wird der Innendurchmesser der Rohrleitung (4) nach dem Diffusor auf einen Innendurchmesser erweitert, der in etwa dem Innendurchmesser der anströmseitig zur Düse (3) angeordneten Rohrleitung (2) entspricht.

Über die Länge der Rohrleitung (4) mit reduziertem Innendurchmesser zwischen Düse (3) und Diffusor kann die Dauer der Kavitation und damit die Einwirkdauer der Kavitation gesteuert werden. Die Länge der Rohrleitung (4) zwischen Düse (3) und Diffusor kann veränderbar ausgestaltet sein, bspw. teleskopartig ausgebildet sein.

Grundsätzlich ist es auch möglich, mehrere Düsen (3), bspw. zwei oder drei Düsen, in einem Rohrleitungssystem hintereinander anzuordnen. Es hat sich jedoch gezeigt, daß eine Verringerung von aufgetretenem Blähschlamm, Schwimmschlamm und/oder Schaum bzw. eine Vermeidung des Auftretens von Blähschlamm, Schwimmschlamm und/oder Schaum durch die erfindungsgemäße Ausgestaltung bereits mit einer Düse (3) erreicht wird.

Die erfindungsgemäße Vorrichtung ist in konstruktiver Hinsicht überraschend einfach. Die erfindungsgemäße Vorrichtung eignet sich mithin äußerst vorteilhaft für einen im wesentlichen wartungsfreien und mithin kostengünstigen Betrieb. Vorteilhaft wird für den Betrieb kein geschultes Personal benötigt. Insofern kann die vorliegende Erfindung vorteilhaft sowohl bei großen als auch bei kleinen Kläranlagen verwendet werden.

Figur 2 zeigt eine schematische Darstellung der Freisetzung von in Schlammaggregaten eingeschlossenen oder anhaftenden Gasblasen, die durch Beaufschlagung mit einem Scherfeld aus den Schlammaggregaten freigesetzt werden. Durch die Freisetzung der in den Schlammaggregaten eingeschlossenen oder anhaftenden Gasblasen wird der Auftrieb der Schlammaggregate wesentlich verringert.

Beim Durchströmen des Abwassers, bevorzugt des Belebtschlamms oder der Mischbiozönose, durch die Düse (3) kommt es weiterhin zu einer Druckabsenkung in dem durchtretenden Abwasser, was eine Ablösung der Gasblasen von den Schlammaggregaten weiter fördert. D.h., das Zusammenwirken der durch die Düse (3) in dem Abwasser induzierten Turbulenz mit der Verringerung des Drucks führt zu einer gewünschten Entgasung der Schlammaggregate bzw. Freisetzung der anhaftenden oder eingeschlossenen Gasblasen.

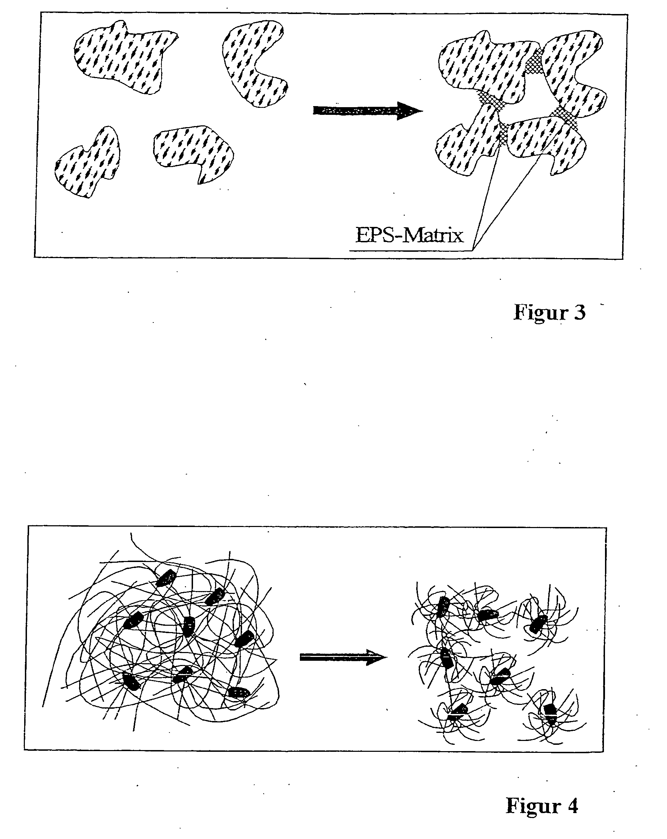

Figur 3 zeigt die durch die Sekretion von extrazellulären polymeren Substanzen (EPS) durch fädige Mikroorganismen bewirkte verbesserte Flockung von zerkleinerten Schlammaggregaten. Durch die in der Düse (3) induzierte Scherbeanspruchung des Abwassers, bevorzugt des Belebtschlamms oder der Mischbiozönose, werden die in dem Abwasser enthaltenen Mikroorganismen einem Streß unterworfen. Der Streß induziert bei Mikroorganismen die Sekretion von EPS. Die Sekretion von EPS führt zu einer flockenartigen Adhäsion von zerkleinerten Schlammaggregaten.

Diese Flockenbildung ist für die gewünschte Sedimentation des Klärschlamms sehr vorteilhaft. Die durch EPS bewirkte Zusammenlagerung von zerkleinerten Schlammaggregaten wirkt einer Bildung von zu kleinen Flocken entgegen, die aufgrund der zu geringen Größe nicht bzw. nicht innerhalb eines gewünschten Zeitraums sedimentieren.

Figur 4 zeigt schematisch die Verkürzung der Länge der Filamente von fädigen Mikroorganismen beim Betrieb der erfindungsgemäßen Vorrichtung. Neben der Verkürzung der Filamentlänge wird auch das gebildete Fadengeflecht aufgebrochen.

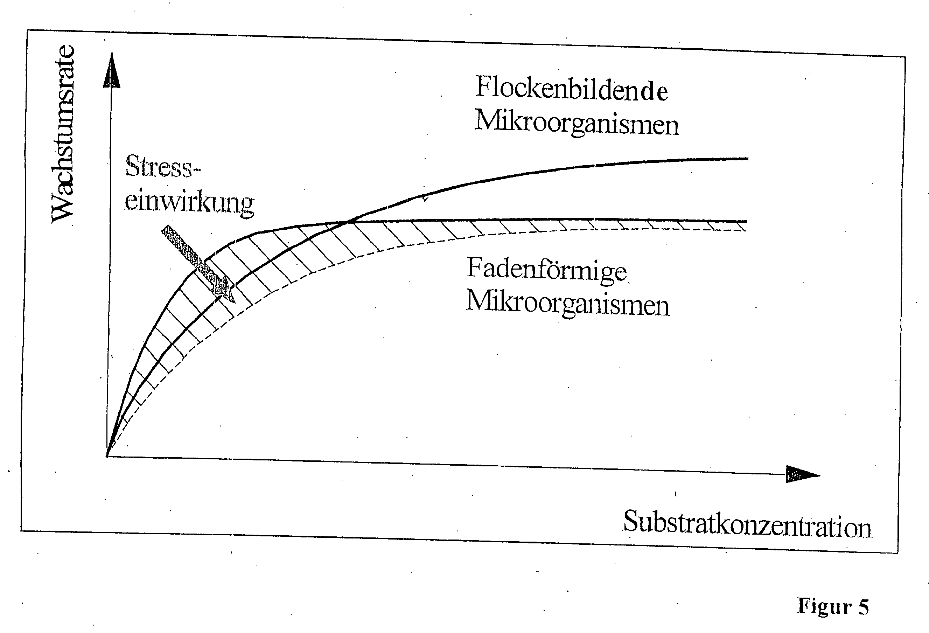

Figur 5 zeigt den Einfluß der Verwendung der erfindungsgemäßen Vorrichtung auf die Wachstumskinetik von flockenbildenden Mikroorganismen und fädigen bzw. fadenförmigen Mikroorganismen. Figur 5 zeigt, daß die Wachstumsrate von flockenbildenden Mikroorganismen beim Betrieb der erfindungsgemäßen Vorrichtung nicht bzw. nicht wesentlich beeinflußt wird.

Hingegen hat der Betrieb der erfindungsgemäßen Vorrichtung einen deutlichen Einfluß auf die Wachstumsrate von fädigen Mikroorganismen. Durch das in der erfindungsgemäßen Vorrichtung angelegte Scherfeld und die vorzugsweise auftretende Kavitation wird das Wachstum von fädigen Mikroorganismen deutlich gegenüber flockenbildenden Mikroorganismen zurückgedrängt. Dies führt zu einer gewünschten Veränderung der relativen Zusammensetzung der Mischbiozönose. Das relative Verhältnis von fädigen Mikroorganismen zu flockenbildenden Mikroorganismen wird erniedrigt.

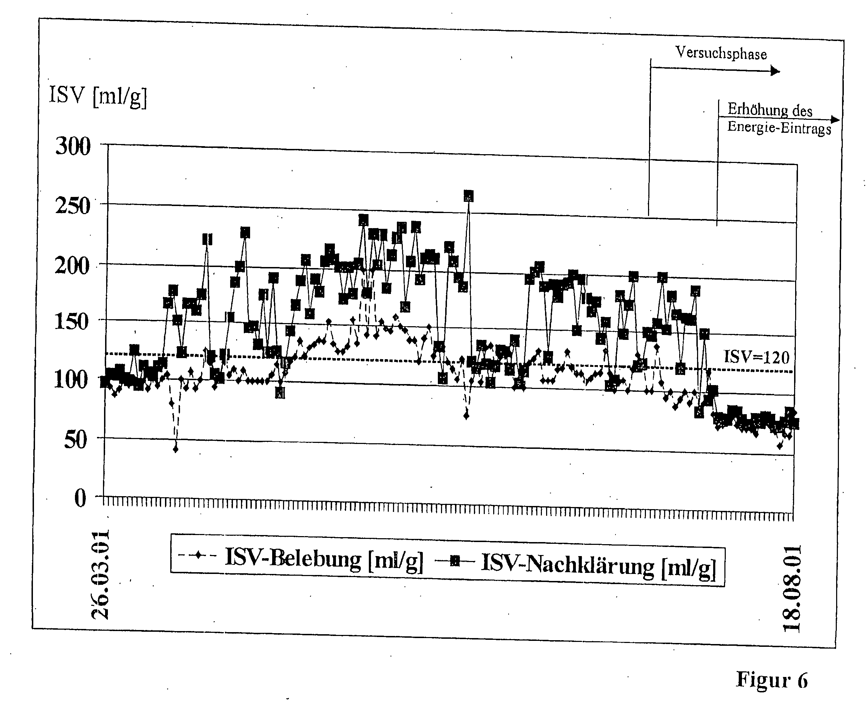

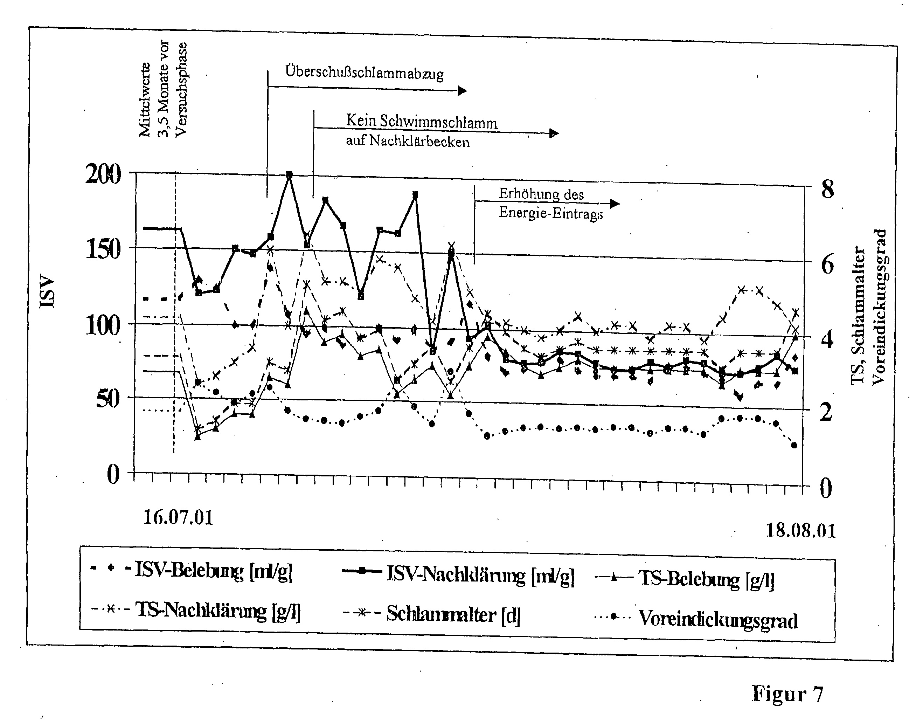

Figur 6 und Figur 7 zeigen Meßkurven, die in einer Versuchskläranlage gemessen wurden.

Die erfindungsgemäße Vorrichtung wurde an einer Kläranlage mit ca. 12.000 angeschlossenen Einwohnergleichwerten betrieben. Das Abwasser im Zulauf dieser Kläranlage war stark mit Abwässern aus der Textilindustrie belastet und führte seit mehr als 10 Jahren zu großen Blähschlamm- und Schwimmschlammproblemen.

Bei der Versuchskläranlage lag der Schlammvolumenindex (ISV) häufig über einem Wert von 120 ml/g. Die sich auf dem Nachklärbecken bildende Schlammdecke wurde bis zu dreimal täglich entfernt. Die Dicke der Schwimmschlammdecke konnte bis zu 5 cm und mehr anwachsen.

Die erfindungsgemäße Vorrichtung wurde direkt in dem Belebungsbecken der Kläranlage angeordnet. Innerhalb der ersten beiden Wochen wurde die erfindungsgemäße Vorrichtung täglich nur 4 Stunden betrieben (Leistung der Pumpe 3,75 kW bei einem Volumenstrom von 10,6 l/s). Der Innendurchmesser der vor der Düse angeordneten Rohrleitung betrug 100 mm, der Innendurchmesser der nach der Düse angeordneten Rohrleitung betrug 50 mm. Der engste Durchmesser der verwendeten Düse betrug 25 mm. Die Düse wies die Geometrie einer Lavaldüse auf.

Vor der Düse war ein Konfusor angeordnet, der den Innendurchmesser der zuführenden Rohrleitung von 100 mm auf 50 mm reduzierte. Abströmseitig von der Düse war in der Rohrleitung eine Drossel angeordnet. Zwischen Düse und Drossel war ein Diffusor angeordnet, der den Innendurchmesser der Rohrleitung von 50 mm wieder auf 100 mm erweiterte. Der Abstand zwischen Düse und Diffusor bzw. die Länge der Rohrleitung mit 50 mm Innendurchmesser betrug 100 cm.

Bereits nach 3 Tagen Versuchsdauer konnte eine deutlich verminderte Fadenlänge von langfädigen Strukturen im Belebtschlamm mikroskopisch beobachtet werden. Dieser Umstand machte sich in einem graduell abnehmenden Schlammvolumenindex (ISV) bemerkbar.

Nach 2 Versuchswochen wurde die erfindungsgemäße Vorrichtung auf Dauerbetrieb umgestellt und der Energieeintrag wurde auf etwa 350 kJ/m³ Mischbiozönose erhöht (Figur 6).

Der Schlammvolumenindex (ISV), der das Volumen des sedimentierten Schlamms von 1 l Inhalt nach 30 Min. Sedimentation, bezogen auf die Ausgangskonzentration beschreibt, ist nach Erhöhung des Energieeintrags auf unter deutlich 100 ml/g gesunken. Bei Werten über 120 ml/g kann es zu erheblichen Problemen der Absetzeigenschaften des Blähschlamms im Nachklärbecken kommen.

Bereits am darauffolgenden Tag konnte eine drastische Längenreduzierung der Filamentlänge von fädigen Mikroorganismen mikroskopisch beobachtet werden. Der Schlammvolumenindex hatte einen Wert von ca. 80 ml/g und hielt diesen Wert über die kommenden Wochen hinweg aufrecht. Dieser Wert liegt deutlich unter dem Schwellenwert von 120 ml/g, ab dem ein Blähschlammverhalten zu erwarten ist. Sowohl im Belebungsbecken als auch im Nachklärbecken kam es zu einer signifikanten Reduktion des Schlammvolumenindexes auf etwa 80 ml/g.

Es hat sich völlig überraschend gezeigt, daß der Energieeintrag in die Mischbiozönose deutlich unter 1.000 kJ/m³ liegen kann, um das Auftreten von Bläh- und Schwimmschlamm zu vermeiden. In Abhängigkeit von dem Anteil an fädigen Mikroorganismen in der Mischbiozönose kann der Energieeintrag beispielsweise in einem Bereich von etwa 200 kJ/m³ Mischbiozönose bis etwa 800 kJ/m³ Mischbiozönose, bevorzugt von etwa 250 kJ/m³ Mischbiozönose bis 600 kJ/m³ Mischbiozönose, weiter bevorzugt von etwa 300 kJ/m³ bis etwa 500 kJ/m³ Mischbiozönose, liegen. Die Geschwindigkeit des durch die Rohrleitung (4) geförderten Abwassers bzw. der Mischbiozönose liegt dabei in einem Bereich von etwa 3 m/s bis 10 m/s, bevorzugt von etwa 4 m/s bis 8 m/s, weiter bevorzugt von etwa 5 m/s bis 7 m/s.

Aus Figur 7 geht hervor, daß es auch bei weiteren Prozeßparametern zu einer Stabilisierung kam, d.h. das erfindungsgemäße Verfahren zu einem stabilen Betrieb der Versuchsanlage führte.

Zu sehen ist deutlich, daß aufgrund der verbesserten Absetzeigenschaften Überschußschlamm abgezogen werden konnte und es damit zu einer Stabilisierung sämtlicher Prozeßparameter kam. Bei Erhöhung des Energieeintrags auf etwa 350 kJ/m³ durch Erhöhung der Pumpleistung erfolgte eine Glättung der erhaltenen Meßkurven, d.h. beim Schlammindex im Belebungsbecken (ISV-Belebung), Schlammindex im Nachklärbecken (ISV-Nachklärung), Trockensubstanz im Belebungsbecken (TS-Belebung), Trockensubstanz im Nachklärbecken (TS-Nachklärung), beim Schlammalter und beim Voreindickungsgrad. Die Mischbiozönose ist deutlich gesundet.

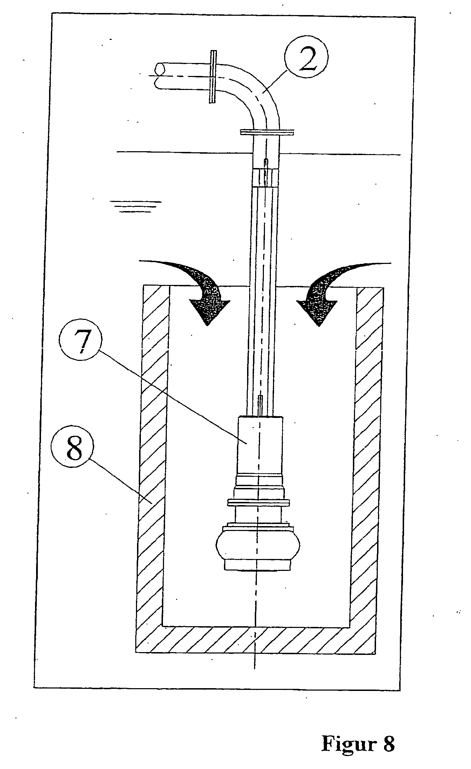

Figur 8 zeigt eine in einem unter der Oberfläche der Mischbiozönose angeordneten Schacht (8) angeordnete Pumpe (7). Der Schacht (8) wird im Sinne der Erfindung als getauchter Schacht (8) bezeichnet. In dem getauchten Schacht (8) ist eine Pumpe (7) angeordnet, die das Abwasser, bevorzugt den Belebtschlamm oder die Mischbiozönose, in die Rohrleitung (2) fördert. Zwischen Pumpe (7) und den Innenwandungen des getauchten Schachts (8) bildet sich eine Entgasungszone aus. In der Entgasungszone werden zumindest teilweise in dem Abwasser bzw. der Mischbiozönose mitgeführte oder enthaltene Gasblasen abgetrennt und freigesetzt, die durch den in Richtung zur Oberfläche offenen Schacht entweichen.

## Patentansprüche

1. Vorrichtung zur Verringerung oder Vermeidung der Bildung von Blähschlamm, Schwimmschlamm und/oder Schaum,
**dadurch gekennzeichnet,**
**daß** die Vorrichtung eine wenigstens eine Verengung (3) aufweisende Rohrleitung (2,4) und eine Einrichtung (1,7) zum Fördern von Abwasser, Klärschlamm oder organischen Substraten durch die Rohrleitung (2,4) aufweist, so daß die Behandlung von fädige und flockenbildende Mikroorganismen-haltigem Abwasser, Klärschlamm oder in Biogasanlagen verwendeten organischen Substraten unter Einwirkung von Kavitation erfolgt, wobei die Verengung eine Düse, die in der Geometrie einer Lavaldüse ausgebildet ist, ist.

2. Vorrichtung gemäß Anspruch 1,
**dadurch gekennzeichnet,**
**daß** der Innendurchmesser der Rohrleitung (2,4) an der Düse (3) am geringsten ist und der Innendurchmesser der Rohrleitung (2) anströmseitig vor der Düse (3) größer ist als der Innendurchmesser der Rohrleitung (4) abströmseitig nach der Düse (3).

3. Vorrichtung gemäß Anspruch 1 oder 2,
**dadurch gekennzeichnet,**
**daß** das Verhältnis des Innendurchmessers der Rohrleitung (2) vor der Düse (3) und des Innendurchmessers der Rohrleitung (4) nach der Düse (3) etwa 5:1 bis etwa 1,2:1 beträgt.

4. Vorrichtung gemäß Anspruch 3,
**dadurch gekennzeichnet,**
**daß** das Verhältnis etwa 3:1 bis etwa 1,5:1, bevorzugt 2:1, beträgt.

5. Vorrichtung gemäß einem der Ansprüche 1 bis 4,
**dadurch gekennzeichnet,**
**daß** die wenigstens eine Düse (3) einen Abschnitt mit konvergierendem und divergierendem Innendurchmesser umfaßt, wobei anströmseitig der Abschnitt mit konvergierendem Innendurchmesser und abströmseitig der Abschnitt mit divergierendem Innendurchmesser angeordnet ist, wobei die Länge des Abschnitts mit konvergierendem Innendurchmesser kürzer ist als die Länge des Abschnitts mit divergierendem Innendurchmesser.

6. Vorrichtung gemäß einem der Ansprüche 1 bis 5,
**dadurch gekennzeichnet,**
**daß** abströmseitig von der Düse (3) in der Rohrleitung (4) eine Drossel (5) angeordnet ist.

7. Vorrichtung gemäß einem der Ansprüche 1 bis 6,
**dadurch gekennzeichnet,**
**daß** die Länge der abströmseitig nach der Düse (3) angeordneten Rohrleitung (4) variabel einstellbar ist.

8. Vorrichtung gemäß einem der Ansprüche 1 bis 7,
**dadurch gekennzeichnet,**
**daß** mehrere Düsen in einer Rohrleitung hintereinander angeordnet sind.

9. Vorrichtung gemäß einem der Ansprüche 1 bis 8,
**dadurch gekennzeichnet,**
**daß** anströmseitig vor der Düse (3) ein Konfusor angeordnet ist.

10. Vorrichtung gemäß einem der Ansprüche 1 bis 9,
**dadurch gekennzeichnet,**
**daß** nach der abströmseitig von der Düse angeordneten Rohrleitung (4), bevorzugt vor der Drossel (5), ein Diffusor angeordnet ist.

11. Vorrichtung gemäß einem der Ansprüche 1 bis 10,
**dadurch gekennzeichnet,**
**daß** die Einrichtung (1,7) zum Fördern des Abwassers, Klärschlamms oder der organischen Substrate eine Pumpe ist.

12. Vorrichtung gemäß Anspruch 11,
**dadurch gekennzeichnet,**
**daß** die Pumpe in einem getauchten Schacht (8) angeordnet ist.

## Claims

1. Device for reducing or avoiding the formation of bulking sludge, scum and/or foam,
**characterized**
**in that** the device has a pipeline (2, 4) having at least one contraction (3) and a means (1, 7) for the conveyance of effluent, sewage sludge or organic substrates through the pipeline (2, 4), so that the treatment of such effluent, sewage sludge or organic substrates used in biogas plants as contain filamentary and flocculating microorganisms takes place under the action of cavitation, the contraction being a nozzle which is designed with the geometry of a Laval nozzle.

2. Device according to Claim 1,
**characterized**
**in that** the inside diameter of the pipeline (2, 4) is the smallest at the nozzle (3), and the inside diameter of the pipeline (2) on the inflow side, upstream of the nozzle (3), is larger than the inside diameter of the pipeline (4) on the outflow side, downstream of the nozzle (3).

3. Device according to Claim 1 or 2,
**characterized**
**in that** the ratio of the inside diameter of the pipeline (2) upstream of the nozzle (3) to the inside diameter of the pipeline (4) downstream of the nozzle (3) is about 5:1 to about 1.2:1.

4. Device according to Claim 3,
**characterized**
**in that** the ratio is about 3:1 to about 1.5:1, preferably 2:1.

5. Device according to one of Claims 1 to 4,
**characterized**
**in that** the at least one nozzle (3) comprises a segment with a converging and a diverging inside diameter, the segment with a converging inside diameter being arranged on the inflow side and the segment with a diverging inside diameter being arranged on the outflow side, the length of the segment with a converging inside diameter being shorter than the length of the segment with a diverging inside diameter.

6. Device according to one of Claims 1 to 5,
**characterized**
**in that** a throttle (5) is arranged in the pipeline (4) on the outflow side of the nozzle (3).

7. Device according to one of Claims 1 to 6,
**characterized**
**in that** the length of the pipeline (4) arranged on the outflow side, downstream of the nozzle (3), can be set variably.

8. Device according to one of Claims 1 to 7,
**characterized**
**in that** a plurality of nozzles are arranged one behind the other in a pipeline.

9. Device according to one of Claims 1 to 8,
**characterized**
**in that** a confuser is arranged on the inflow side, upstream of the nozzle (3).

10. Device according to one of Claims 1 to 9,
**characterized**
**in that** a diffuser is arranged downstream of the pipeline (4) arranged on the outflow side of the nozzle, preferably upstream of the throttle (5).

11. Device according to one of Claims 1 to 10,
**characterized**
**in that** the means (1, 7) for conveying the effluent, sewage or the organic substrates is a pump.

12. Device according to Claim 11,
**characterized**
**in that** the pump is arranged in a submerged well (8).

## Revendications

1. Dispositif pour diminuer ou éviter la formation de boue foisonnante (bulking), de boue surnageante et/ou de mousse, **caractérisé en ce que** le dispositif présente un conduit (2,4) présentant au moins un rétrécissement (3) et une installation (1,7) pour pousser les effluents, les boues résiduaires ou les substrats organiques au travers du conduit (2,4), de telle sorte que le traitement des effluents filamenteux et floculants contenant des microorganismes, des boues résiduaires ou des substrats organiques utilisés dans des installations à biogaz s'effectue sous l'effet d'une cavitation, sachant que le rétrécissement est une buse qui est conformée avec la géométrie d'une buse à effet Laval.

2. Dispositif selon la revendication 1, **caractérisé en ce que** le diamètre intérieur du conduit (2,4) au niveau de la buse (3) est le plus petit et le diamètre intérieur du conduit (2) du côté amont avant la buse (3) est plus grand que le diamètre intérieur du conduit (4) du côté aval après la buse (3).

3. Dispositif selon la revendication 1 ou la revendication 2, **caractérisé en ce que** le rapport du diamètre intérieur du conduit (2) avant la buse (3) au diamètre intérieur du conduit (4) après la buse (3) s'élève à une valeur d'environ 5/1 à environ 1,2/1.

4. Dispositif selon la revendication 3, **caractérisé en ce que** le rapport s'élève à une valeur d'environ 3/1 à environ 1,5/1, de préférence de 2/1.

5. Dispositif selon l'une quelconque des revendications 1 à 4, **caractérisé en ce qu'**au moins une buse (3) comprend une section avec un diamètre intérieur convergent et un diamètre intérieur divergent, sachant que la section avec le diamètre intérieur convergent est disposée du côté amont et la section avec le diamètre intérieur divergent est disposée du côté aval, sachant que la longueur de la section avec le diamètre intérieur convergent est plus courte que la longueur de la section avec le diamètre intérieur divergent.

6. Dispositif selon l'une quelconque des revendications 1 à 5, **caractérisé en ce qu'**une valve papillon (5) est disposée en aval de la buse (3) dans le conduit (4).

7. Dispositif selon l'une quelconque des revendications 1 à 6, **caractérisé en ce que** la longueur du conduit (4) disposé en aval après la buse (3) peut être ajustée de manière variable.

8. Dispositif selon l'une quelconque des revendications 1 à 7, **caractérisé en ce que** plusieurs buses sont disposées dans un conduit l'une derrière l'autre.

9. Dispositif selon l'une quelconque des revendications 1 à 8, **caractérisé en ce qu'**un mélangeur est disposé en amont devant la buse (3).

10. Dispositif selon l'une quelconque des revendications 1 à 9, **caractérisé en ce qu'**un diffuseur est disposé, après le conduit (4) situé en aval de la buse, de préférence devant la valve papillon (5).

11. Dispositif selon l'une quelconque des revendications 1 à 10, **caractérisé en ce que** l'installation (1,7) pour pousser les effluents, les boues résiduaires ou les substrats organiques est une pompe.

12. Dispositif selon la revendication 11, **caractérisé en ce que** la pompe est disposée dans un puits immergé (8).
